# EUROPÄISCHE PATENTSCHRIFT

(11) **EP 1 091 694 B1**
(45) Veröffentlichungstag und Bekanntmachung des Hinweises auf die Patenterteilung: **07.09.2005**
(21) Anmeldenummer: 99924650.7
(22) Anmeldetag: 22.06.1999
(51) Int. Cl.: A61B 17/00

(54) **VORRICHTUNG ZUM EINBRINGEN VON FIBRINKLEBER IN EINEN STICHKANAL**
DEVICE FOR INTRODUCING FIBRIN ADHESIVE INTO A PUNCTURE CHANNEL
DISPOSITIF POUR INTRODUIRE DE LA COLLE A LA FIBRINE DANS UN CANAL DE PONCTION

(30) Priorität: 02.07.1998 CH 141598
(43) Veröffentlichungstag der Anmeldung: 18.04.2001
(73) Patentinhaber: Baxter Healthcare SA, 8304 Wallisellen (CH); Baxter International Inc., Deerfield, IL 60015 (US)
(72) Erfinder: ILLI, Oscar, E., CH-8603 Schwerzenbach (CH)
(74) Vertreter: Schneider Feldmann AG Patent- und Markenanwälte
(86) Internationale Anmeldenummer: PCT/CH1999/000273
(87) Internationale Veröffentlichungsnummer: WO 2000/001305

(56) Entgegenhaltungen:
- WO-A-94/28798
- US-A- 5 676 689
- US-A- 5 728 132

## Beschreibung

Die vorliegende Erfindung betrifft eine Vorrichtung zur Einbringung von Zweikomponenten Fibrinkleber in einen Stichkanal in die Nähe einer arteriellen oder venösen Punktionsstelle gemäss dem Oberbegriff des Patentanspruches 1.

Viele human- und veterinärmedizinische Eingriffe verlangen die Punktierung von Gefässen. Insbesondere bei perkutaner transluminarer Koronarangioplastie (PTCA), Herzoperationen und Herzkatheterisierungen müssen die punktierten Gefässe mit aller Vorsicht wieder verschlossen werden. In den meisten aller Fälle erfolgt dies mittels direkter Kompression von bis zu 1 Stunde und einem Druckverband, der bis zu 24 Stunden angelegt sein muss und eine 1 - 2 tägige Hospitalisation verlangt. Folglich besteht das Bestreben eine Lösung zu finden, die zu einem schnelleren und sichereren Verschluss der Punktionsstelle führt.

Anlässlich eines Meetings der American Heart Association vom 17.11.1992 in New Orleans wurde eine Methode unter der Bezeichnung Vasoseal vorgestellt. Bei dieser Methode wurden jeweils zwei Kollagenpfropfen aus bovinem Kollagen in den Stichkanal bis an die Punktionsstelle eingestossen. Am erwähnten Meeting wurde festgehalten, dass neben der eher seltenen Abstossungsreaktion des körperfremden Kollagens diverse weitere Nachteile bzw. Risiken bestehen. So wurde festgehalten, dass dieses System in vielen Fällen ineffektiv ist und eine gewisse Emboliegefahr besteht. Bei rund 46% aller Fälle bildeten sich Hämatome in der Grössenordnung von 2-6 cm. Bis zur vollständigen Resorption des bovinen Kollagens vergehen Wochen bis Monate.

Die Methode führt zudem zu einer vergrösserten Narbenbildung, die eine Ultraschallkontrolle erschwert. Schliesslich wurde die Hospitalisierung nicht überflüssig, aber zumindest verkürzt. Eines der wesentlichsten Probleme besteht jedoch in der Handhabung, d.h. die Einführung des Kollagenpfropfens in den Stichkanal. Da nacheinander zwei Kollagenpfropfen in den Stichkanal eingedrückt werden müssen, ist beispielsweise die Eindringtiefe für den Anwender schwer feststellbar. Werden die Kollagenpfropfen zu tief eingestossen, so kann der Kollagenpfropfen durch die Punktionsstelle in das Gefäss eingeschoben werden, was zu einem Verschluss führen würde oder das Gefäss selber zudrückt. Eine neue Methode, um solche Punktionsstellen schnell, zuverlässig und ohne die nachteiligen Kollagenpfropfen zu verschliessen, hat der Erfinder in der WO 94/28798 offenbart.

Aus der FR-A-2378528 ist ein doppelluminarer Katheter bekannt zur Durchführung der Hämodialyse. Dabei wird das zu dialysierende Blut durch den exterioren Lumen entnommen und durch den interioren Lumen dialysiertes Blut wieder zugeführt. Das äussere Lumen umgibt dabei das innere Lumen distanziert, und beide Lumen werden während der Dialyse in das Gefäss des Patienten eingeführt.

Gemäss der EP-A-0'482'350 wird ein Gerät vorgeschlagen, das normalerweise über den Guidewire geschoben wird und mit dem ein Pfropfen in den Stichkanal eingeschoben wird. Obwohl der Dilator einen gering grösseren Durchmesser als die Kanüle aufweist, in die der Guidewire geführt wird, ist der Widerstand, den der Kardiologe wahrnimmt, beim Einführen gering, so dass die Gefahr äusserst realistisch ist, dass die äussere Kanüle des Gerätes in das zu versiegelnde Gefäss eingestossen wird. Somit wird dann der Pfropfen statt in den Stichkanal in das Gefäss plaziert. Diese Gefahr wurde erkannt, und es wird daher in derselben Anmeldung vorgeschlagen, mittels einer Nadelklemme die Eindringtiefe der Nadel abzumessen, wenn der Guidewire eingeführt wird.

Es hat sich gezeigt, dass bei der Verwendung von aus Bluteiweiss gewonnenem, körpereigenem Blutgerinnungsmittel in der Form von Zweikomponenten Fibrinkleber, dessen Komponenten man im Moment der Zuführung vermischt und diese Mischung während oder direkt im Anschluss an einen intravasalen Eingriff in den Stichkanal möglichst gefässnah einbringt, ein optimaler Gefässverschluss entsteht. Histologische Untersuchungen haben diesen Sachverhalt bestätigt.

Die Lösung, den Stichkanal mit einem Zweikomponenten-Fibrinkleber zu versiegeln, hat, wie verschiedene klinische Versuche zeigten, eine hohe funktionelle Sicherheit. Trotz dem Einsatz von Blutverdünnungsmittel konnte eine einwandfreie Versiegelung erzielt werden.

Während bei den ersten klinischen Erprobungen praktisch keine Fehlmanipulationen festgestellt wurden, zeigte es sich, dass die hohe funktionelle Sicherheit dazu führte, dass bei einer weiteren Versuchsserie sehr schnell gearbeitet wurde und entgegen den Weisungen die Vorsichtsmassnahmen nicht eingehalten wurden. Entsprechend ergaben sich teilweise ungenügende Versiegelungen, die einer Nachbehandlung bedurften. Leider wurde die Versiegelung entweder zu tief vorgenommen, wodurch ein Teil des Fibrinklebers in das Gefäss gelangte oder die Versiegelung wurde zu weit von der Gefässwand entfernt vorgenommen, wodurch nicht abgebender Fibrinkleber aussen an der Kanülenwand entlang auf den Einstichkanal hinausdrang.

Es ist folglich die Aufgabe der vorliegenden Erfindung, eine Vorrichtung zu schaffen, mittels der Fibrinkleber in einen Stichkanal möglichst exakt in die Nähe einer arteriellen oder venösen Punktionsstelle gebracht werden kann.

Diese Aufgabe löst eine Vorrichtung, die eine Versiegelungskanüle umfasst, die von einer Arbeitskanüle von oben nach unten axial durchsetzt wird, wobei die Arbeitskanüle, die zur intravasalen Einführung eines Instrumentariums in ein Gefäss dient, von der Versiegelungskanüle distanziert umgeben ist, so dass der Fibrinkleber von einem Anschlussstutzen zumindest einer radial gerichteten Austrittsöffnung in der Versiegelungskanüle zwischen dieser und der Arbeitskanüle geführt ist, mit den Merkmalen des Patentanspruches 1.

Weitere vorteilhafte Ausgestaltungsformen der Vorrichtung gehen aus den abhängigen Ansprüchen hervor, und deren Bedeutung und Vorteile sind in der nachfolgenden Beschreibung dargelegt.

In der Zeichnung ist eine bevorzugte Ausführungsform des Erfindungsgegenstandes sowie eine Lösung gemäss dem Stand der Technik dargestellt und anhand der nachfolgenden Beschreibung erläutert. Es zeigt:
- Fig. 1: die erfindungsgemässe Vorrichtung in der Einführungslage und
- Fig. 2: in der Versiegelungslage beide Male im Schnitt, während
- Fig. 3: die Situation nach Fig. 1 und
- Fig. 4: die Situation nach Fig. 2 in der Ansicht darstellen.
- Fig. 5: stellt einen Radialschnitt entlang der Linie B-B in Fig. 4 dar.
- Fig. 6: ist wiederum ein Axialschnitt entlang der Linie A-A in Fig. 5.
- Fig. 7.1 - 7.6: zeigt die verschiedenen Schritte der Verwendung der erfindungsgemässen Vorrichtung.

Die generelle Ausführungsform der Vorrichtung gemäss den Figuren besteht lediglich aus drei zusammenfügbaren Teilen. Mit dem Bezugszeichen 1 ist die Arbeitskanüle, in der Fachsprache auch Worksheath genannt, bezeichnet. Die Arbeitskanüle selber ist lediglich ein an beiden Enden offenes, zylindrisches Röhrchen aus Kunststoff. Dessen vorderes Ende 1' dient der Einführung der Kanüle durch die Punktionsstelle in das geöffnete Blutgefäss. Die Arbeitskanüle ist relativ dünnwandig und weist demzufolge eine gewisse Biegeelastizität auf. In dieser Ausgestaltungsform ist die. Arbeitskanüle 1 am anderen Ende, dem rückwärtigen Ende, fest mit einem Medizinalkupplungsstück 3 verbunden. Das eigentliche Medizinalkupplungsstück 3 kann beispielsweise eine bekannte Luerlock-Kupplung sein. Auf dem eigentlichen Medizinalkupplungsstück 3 ist eine exakt gearbeitete, formschlüssig und dichtend passende Muffe 33' angeformt, in der die Arbeitskanüle gehalten ist. Die Arbeitskanüle 1 verläuft in Längsrichtung axial durch eine Versiegelungskanüle 2 und ragt unten ein Stück weit aus der Versiegelungskanüle heraus. Die Versiegelungskanüle 2 ist selber auch wieder in der Form eines konzentrischen Röhrchens gestaltet, doch ist dessen Innendurchmesser, vorzugsweise jedoch auch dessen Aussendurchmesser, von oben nach unten abnehmend, d.h. der Innendurchmesser nimmt von der Seite, an der das Medizinalkupplungsstück 3 eingeschoben wird, bis zum unteren Ende, wc die Arbeitskanüle aus der Versiegelungskanüle 2 heraustritt, ab.

Über die gesamte Länge, auf der die Arbeitskanüle 1 von der Versiegelungskanüle 2 konzentrisch umgeben ist, verbleibt somit zwischen der Arbeitskanüle und der Versiegelungskanüle ein Hohlraum 7. Am oberen Ende weist die Versiegelungskanüle 2 eine verstärkte Manschette 4 auf, die eine erheblich dickere Wandstärke als die Wandstärke der Versiegelungskanüle 2 hat. Im Bereich der verstärkten Manschette 4 mündet ein Anschluss bzw. Versiegelungsstutzen 5 in die Versiegelungskanüle 2. Durch diesen Versiegelungsstutzen 5 lässt sich ein Zweikomponenten-Fibrinkleber in den Hohlraum 7 zwischen Arbeitskanüle und Versiegelungskanüle einführen.

Der Fibrinkleber kann aus dem Hohlraum 7 nur durch die Austrittsöffnungen 6 am unteren Bereich der Versiegelungskanüle 2 austreten. Damit nicht unbeabsichtigterweise Fibrinkleber in das Blutgefäss eindringt, sind die Austrittsöffnungen 6 mindestens annähernd radial nach aussen gerichtet. Selbstverständlich ist unter radial nicht nur die im streng geometrischen Sinne verstandene Richtung gemeint. Vielmehr soll hiermit lediglich zum Ausdruck gebracht werden, dass die Ausströmungsrichtung nicht axial ist. Selbstverständlich wird die Funktion der Vorrichtung durch eine einzige Austrittsöffnung bereits sichergestellt, doch wird man vorzugsweise mehrere am Umfang verteilte Austrittsöffnungen 6 vorsehen. Auch ist die Ausgestaltungsform der Austrittsöffnungen 6 prinzipiell frei gestaltbar. Aus fertigungstechnischen Gründen wird man diese jedoch vorzugsweise in der Gestalt von mehreren am Umfang verteilten Längsschlitzen formen.

Obwohl bevorzugterweise der Versiegelungsstutzen 5 einstückig direkt im Bereich der verstärkten Manschette an der Versiegelungskanüle 2 angeformt ist, ist es selbstverständlich auch möglich, den Anschluss bzw. Versiegelungsstutzen getrennt zu fertigen und nachträglich beispielsweise dank einem Gewinde mit der Versiegelungskanüle zu verbinden. Statt der Schraubverbindung ist selbstverständlich auch eine Schweiss- oder Klebverbindung denkbar. Für die Vermischung der beiden Komponenten des Zweikomponenten-Fibrinklebers sind auf dem Markt bereits Mischorgane erhältlich. Aus Kostengründen wird man folglich den Anschlussstutzen 5 so dimensionieren, dass in denselben ein bereits erhältliches Mischorgan einschiebbar ist.

Wie bereits erwähnt ist die Wanddicke der Arbeitskanüle 1 sehr gering. Sie beträgt vorzugsweise lediglich einige Zehntelmillimeter. Auch der konzentrisch um die Arbeitskanüle 1 zwischen deren Aussenwand und der Innenwand der Versiegelungskanüle 2 verbleibende Hohlraum 7 ist äusserst klein dimensioniert. Da durch die Arbeitskanüle 1 das gesamte chirurgische Instrumentarium ein- und ausgestossen werden muss, ist es von Vorteil, Mittel vorzusehen, die bewirken, dass dieser Hohlraum 7 durchgehend offen bleibt.

Hierzu sind an der Innenwand der Versiegelungskanüle 2 Stützrippen 9, die vorzugsweise axial verlaufen, angeordnet.

Die Stützrippen 9 ergeben auch eine Versteifung der ebenfalls dünnwandigen Versiegelungskanüle 2. Hiermit wird auch die Gefahr, dass eine geringfügige Kontraktion des Muskelgewebes, durch das die Versiegelungskanüle hindurchverläuft, zu einer Deformation der Versiegelungskanüle 2 führt, welche den Hohlraum 7 verschliessen könnte, ausgeschlossen. Auf diese Weise ist der erforderliche Durchgang für den Fibrinkleber auf jeden Fall gesichert.

Das wesentliche Bauteil der erfindungsgemässen Vorrichtung ist das besonders gestaltete Medizinalkupplungsstück 3. Dieses Medizinalkupplungsstück 3 ist fest mit der Arbeitskanüle 1 verbunden. Bewegungen, die das Medizinalkupplungsstück 3 ausführt, werden somit direkt auf die Arbeitskanüle 1 übertragen. Das Medizinalkupplungsstück 3 hat eine zentrale, sie längs durchsetzende Einführungsöffnung 30, die am distalen Ende erweitert ist und am proximalen Ende in ein dichtendes Rohrstück 38 übergeht, das dichtend in die zuvor beschriebene Manschette 4 am distalen Ende der Versiegelungskanüle 2 passt. Das dichtende Rohrstück 38 wird distanziert von einer daran einstückig angeformten Muffe 33 umgeben, wobei die Muffe 33' ein Innengewinde 33 aufweist. Dank diesem Gewinde 33 lässt sich das Medizinalkupplungsstück 3 auf die Manschette 4 aufschrauben, die an ihrem distalen Ende ein Aussengewinde 20 aufweist. Schräg zur zentralen Achse ist am Medizinalkupplungsstück 3 ein Kontrollstutzen 31 angeformt. Dieser Stutzen 31 kommuniziert mit der Einführungsöffnung 30 des Medizinalkupplungsstück 3. Der Kontrollstutzen 31, der mindestens annähernd in einem gleichen Winkel zur zentralen Achse geneigt nach oben gerichtet ist, wie der zuvor beschriebene Anschluss- oder Versiegelungsstutzen 5 an der Versiegelungskanüle 2, hat an seinem Ende eine Luerlock-Kupplung 32.

Das distale Ende der Einführungsöffnung 30 wird mittels einer Durchführungsdichtung 35 verschlossen. Eine Fixierungskappe 36 hält die Durchführungsdichtung 35 fest fixiert auf dem distalen Ende des Medizinalkupplungsstücks 3. Die Fixierungskappe 36 hat ein Einführungsloch 37 zur Durchführung der intraluminar zu verwendenden Elemente, wie beispielsweise den Guidewire, verschiedene Katheter insbesondere auch Ballonkatheter sowie Stents. Bei der Durchführungsdichtung 35 handelt es sich insbesondere um eine hämostatische Dichtung bzw. um ein hämostatisches Ventil, wie es aus diversen Einführungsbestecken bekannt ist.

Letztlich zeigt das Medizinalkupplungsstück 3 als Besonderheit ein radial nach aussen und unten gerichtetes Abdeckschild 39 sowie einen ebenfalls nach aussen gerichteten und nach unten geneigten Betätigungshebel 34. Auch diese beiden Teile 39,34 sind einstückig mit dem Medizinalkupplungsstück 3 verformt.

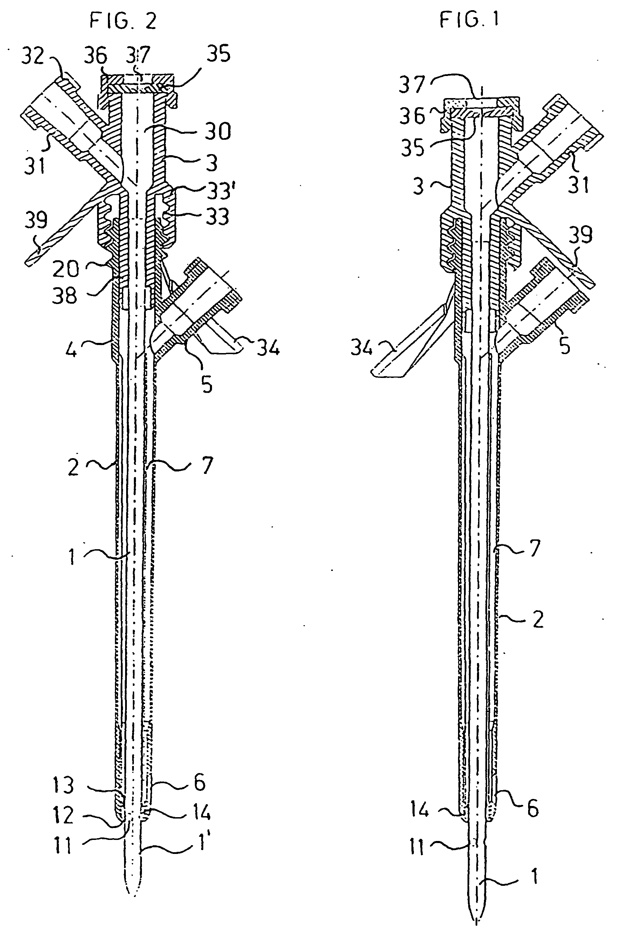

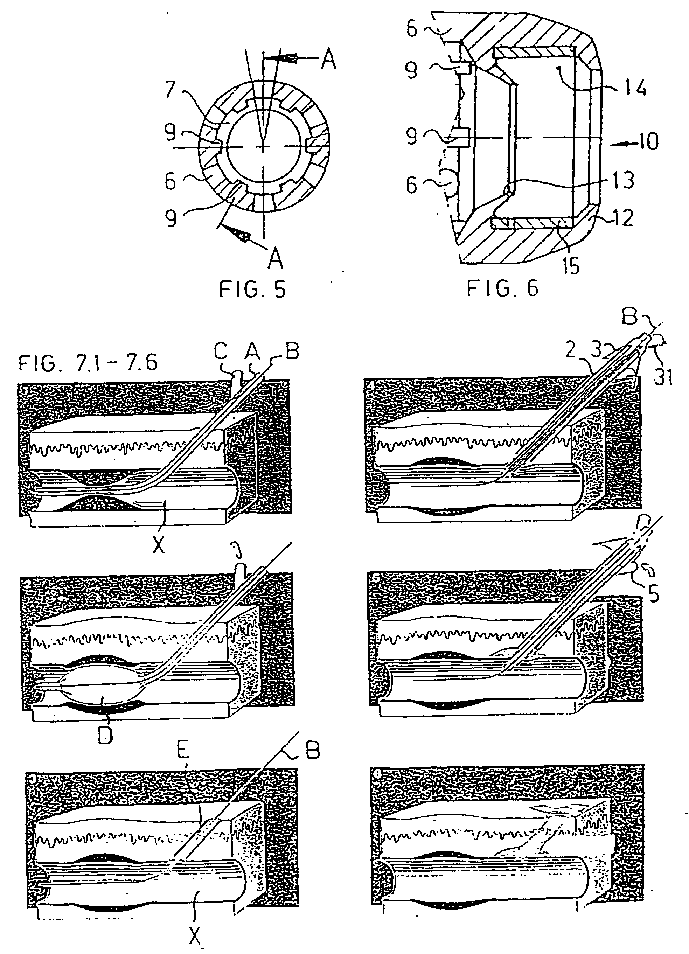

Der Sinn des speziell ausgestalteten Medizinalkupplungsstückes 3 ergibt sich erst aus dem Zusammenwirken dieses Elementes mit der daran fest angeordneten Arbeitskanüle 1 und der dazu beweglich gehaltenen Versiegelungskanüle 2. Die Versiegelungskanüle 2 hat nicht nur die bereits beschriebenen Austrittsöffnungen 6, sondern in proximaler Richtung von diesen Öffnungen 6 aus gesehen eine Durchtrittsöffnung 10, durch welche die Arbeitskanüle 1 mit ihrem proximalen Ende 1' nach aussen und innen verschiebbar geführt ist. Zwischen der eigentlichen Durchtrittsöffnung 10 und den Austrittsöffnungen 6 weist die Versiegelungskanüle 2 zwei nach innen gerichtete ringförmige Dichtlippen 12 und 13 auf. Zwischen der proximalen Dichtlippe 12 und der distalen Dichtlippe 13 verbleibt so eine ringförmige Dichtkammer 14. Diesbezüglich wird insbesondere auf die Figur 6 verwiesen. Wie in den Figuren 1 und 2 sowie 3 und 4 deutlich erkennbar, ist im proximalen Endbereich der Arbeitskanüle 1 mindestens eine vorzugsweise jedoch mehrere Einlassöffnungen 11 vorgesehen. Diese liegen allesamt auf einer Höhe.

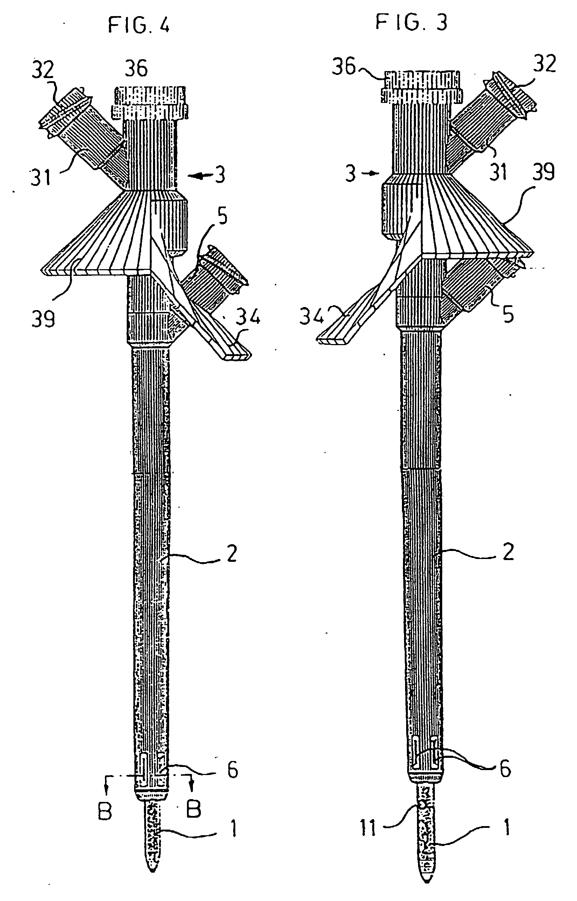

In der Lage nach den Figuren 1 und 3 ist die Arbeitskanüle 1 so weit nach aussen geschoben, dass die Einlassöffnungen 11 ausserhalb der Versiegelungskanüle 2 liegen, während sie in den Figuren 2 und 4 innerhalb derselben liegen.

Nachfolgend wird nun die Verwendung der erfindungsgemässen Vorrichtung unter Hinweis auf die Figuren 7.1 bis 7.6 erläutert. In der Figur 7.1 erkennt man das mit X bezeichnete Lumen, hier ein verengtes Gefäss, beispielsweise eine Arterie, in die mittels einem Einführungsbesteck A ein Guidewire B eingeführt ist. Über diesen Guidewire B wird in den Bereich der Verengung ein Ballonkatheter D geschoben und über den Stutzen C aufgeblasen. Aus der nun korrekt geweiteten Arterie wird über den Guidewire B sowohl der Katheter als auch das Einführungsbesteck A entfernt. Lediglich der Guidewire B wird am Ort belassen. Der Einstichkanal E ist nun offen, und das Blut strömt durch den Einstichkanal E aus. Nun wird über den Guidewire B die erfindungsgemässe Vorrichtung eingeschoben. Der Guidewire B durchsetzt dabei die Arbeitskanüle 1 sowie das Medizinalkupplungsstück 3 und durchstösst das hämostatische Ventil 35 und tritt durch die Öffnung 37 des Fixierungsdeckels 36 aus. In dieser Einführungsposition befindet sich die Vorrichtung in der Lage, wie in den Figuren 1 und 3 dargestellt. Die Arbeitskanüle 1 ragt dabei so weit aus der Versiegelungskanüle 2 hinaus, dass die Einlassöffnungen 11 frei daliegen. Während des Einschiebens verschliesst das elastische Gewebe des Patienten die Einlassöffnungen 11 in der Arbeitskanüle 1 weitgehend. Folglich tritt kaum Blut in die Arbeitskanüle 1. Diese Einführungsposition ist in der Figur 7.4 dargestellt. Im Prinzip ist es nicht erwünscht, dass der Arzt die Versiegelungskanüle 2 in die Arterie stösst. Dies ist mittels der neuen Vorrichtung problemlos vermeidbar. Schiebt der Arzt die Vorrichtung langsam vorwärts, so tritt erst die Arbeitskanüle 1 in die Arterie X ein, bis das proximale Ende der Versiegelungskanüle 2 an der Arterienwand anliegt. Dies wird dem Arzt dadurch angezeigt, dass nun die Einlassöffnungen 11 in der Arbeitskanüle 1 in der Arterie liegen und somit durch diese Einlassöffnungen 11 das Blut in die Arbeitskanüle 1 hochsteigt und beim Kontrollstutzen 31 hinausströmt.

Drückt der Arzt trotz dieser korrekten Position weiter, so wird er, bevor die Versiegelungskanüle in die Arterie eindringt, die Arterie praktisch erst zudrücken oder zumindest teilweise zudrücken, so dass der pulsierende Blutstrahl reduziert wird. So erhält der Arzt eine zweite Information.

Sobald das Blut wie erwähnt aus den Kontrollstutzen 31 strömt, verdreht der Arzt das Medizinalkupplungsstück 3 mittels dem Betätigungshebel 34 um einen gewissen Winkel von beispielsweise 180 Grad. Hierdurch wird das Medizinalkupplungsstück 3 dank den beiden Gewinden 20,33 relativ zur Versiegelungskanüle 2 nach oben verschoben. Entsprechend wird die Arbeitskanüle 1 um dieses Mass in die Versiegelungskanüle 2 hineingezogen. Diese Situation ist in der Figur 7.5 dargestellt. Durch die Verdrehung des Medizinalkupplungsstückes 3 zur Versiegelungskanüle 2 wird das Abdeckschild 39 am Medizinalkupplungsstück 3 weggedreht und gibt so den Einlass des Anschluss- bzw. Versiegelungsstutzens 5 frei. Nun kann am Versiegelungsstutzen 5 ein Mittel angeschlossen werden, um entsprechend ein Material zur Versiegelung des Einstichkanals zu injizieren. Dank des Abdeckschilds 39 ist es nicht möglich, vor der korrekten Plazierung das Versiegelungsmaterial an den Versiegelungsstutzen 5 anzuschliessen.

Durch die erwähnte Verdrehung des Medizinalkupplungsstückes 3 zur Versiegelungskanüle 2 gelangen die erwähnten Einlassöffnungen 11 in den zuvor beschriebenen inneren Bereich der Versiegelungskanüle 2. Dieser Bereich ist die Dichtkammer 14, die entsprechend so dimensioniert ist, dass der Abstand zwischen der proximalen Dichtlippe 12 und der distalen Dichtlippe 13 grösser ist als der Durchmesser der Einlassöffnungen 11.

Obwohl die zuvor beschriebenen Mittel eine eindeutige Positionierung der Versiegelungskanüle 2 direkt an der Durchstossstelle zur Arterie verbringbar ist, wird oftmals trotzdem noch der Wunsch vorhanden sein, dass die richtige Positionierung auch noch sichtbar ist.

Dies wurde bei der vorliegenden Erfindung dadurch erreicht, dass man in der Dichtkammer 14 zwischen den beiden Dichtlippen 12 und 13 einen Ring 15 angeordnet hat. Dieser Ring 15 ist so klein dimensioniert, dass er nur in der Figur 6 erkennbar ist. Der Ring 15 kann im Prinzip metallisch sein oder auch nur aus einer Beschichtung bestehen, die radiologisch erkennbar ist. Auch ein Kunststoffring, der aus einem Material gefertigt ist, dem radiologisch erkennbare Substanzen zugemischt worden sind, ist denkbar. Insbesondere verschiedene Bariummischungen sind als geeignet bekannt. Insbesondere metallische Ringe sind geeignet zur sonographischen Ermittlung der Lage des proximaler Endes der Versiegelungskanüle.

Trotz der hohen mit dem erfindungsgemässen Gerät erzielbaren Sicherheit bei der Versiegelung von Einstichkanälen, wird diese erhöhte Sicherheit kaum mit einem Mehrpreis der Vorrichtung erreicht.

## Patentansprüche

1. Vorrichtung zur Einbringung von Zweikomponenten-Fibrinkleber in einen Stichkanal in die Nähe einer arteriellen oder venösen Punktionsstelle, wobei diese eine Versiegelungskanüle (2) umfasst, die von einer Arbeitskanüle (1) von oben nach unten axial durchsetzt wird, die zur intravasalen Einführung eines Instrumentariums in ein Gefäss dient, wobei die Arbeitskanüle (1) von der Versiegelungskanüle (2) distanziert umgeben ist, so dass der Fibrinkleber von einem Anschlussstutzen (5) zu mindestens einer radial gerichteten Austrittsöffnung (6) in der Versiegelungskanüle zwischen dieser und der Arbeitskanüle geführt ist, und wobei die Versiegelungskanüle (2) am distalen Ende mit einer Manschette oder Wandverdichtung (4) seitlich einstückig angeformt ist, **dadurch gekennzeichnet, dass** die Manschette (4) am distalen Bereich ein Aussengewinde (20) aufweist, auf dem ein Medizinalkupplungsstück (3) mit Innengewinde schraubbar gehalten ist, welches eine distale Einführungsöffnung (30) aufweist und fluchtend fest verbunden mit der Arbeitskanüle ist, die am proximalen Ende Einlassöffnungen (11) aufweist, welche durch eine Relativbewegung des Medizinalkupplungsstückes (3) zur Manschette (4) in eine Verschliesslage in die Versiegelungskanüle (2) hinein bewegbar sind

2. Vorrichtung nach Anspruch 1, **dadurch gekennzeichnet, dass** das Medizinalkupplungsstück (3) einen seitlichen Kontrollstutzen (31) aufweist.

3. Vorrichtung nach Anspruch 2, **dadurch gekennzeichnet, dass** am Kontrollstutzen (31) eine Luerlock-Kupplung (32) angeordnet ist.

4. Vorrichtung nach Anspruch 1, **dadurch gekennzeichnet, dass** am Medizinalkupplungsstück (3) ein Abdeckschild (39) so abstehend angebracht ist, dass dieses den Anschlussstutzen (5) überdeckt, wenn die Einlassöffnungen (11) in der Öffnungslage sind, während es den Anschlussstutzen (5) freigibt, wenn die Einlassöffnungen (11) in der Schliessstellung sind.

5. Vorrichtung nach Anspruch 1, **dadurch gekennzeichnet, dass** das Medizinalkupplungsstück (3) ein Innengewinde (38) aufweist, das mit einem Aussengewinde (20) auf der Manschette (5) klemmt, so dass durch Verdrehung des Medizinalkupplungsstückes (3) zur Manschette (4) die Einlassöffnungen (11) in die Schliesslage innerhalb der Versiegelungskanüle (2) bzw. Öffnungslage ausserhalb der Versiegelungskanüle bewegbar sind.

6. Vorrichtung nach Anspruch 1, **dadurch gekennzeichnet, dass** am Medizinalkupplungsstück (3) ein Betätigungshebel (34) angeformt ist.

7. Vorrichtung nach Anspruch 1, **dadurch gekennzeichnet, dass** am distalen Ende des Medizinalkupplungsstückes (3) eine Durchführungsdichtung (35) mit einer Fixierung (36) gehalten ist.

8. Vorrichtung nach Anspruch 7, **dadurch gekennzeichnet, dass** die Durchführungsdichtung (35) gleichzeitig ein Hämostaseventil bildet.

9. Vorrichtung nach Anspruch 1, **dadurch gekennzeichnet, dass** das proximale Ende der Versiegelungskanüle zwei voneinander mindestens um den Durchmesser der Einlassöffnungen (11) distanzierte radial und nach innen gerichtete, dichtend an der Arbeitskanüle (1) anliegende ringförmige Dichtlippen (12,13) aufweist, die eine ringförmige Dichtkammer (14) bilden.

10. Vorrichtung nach Anspruch 1, **dadurch gekennzeichnet, dass** in der Dichtkammer (14) ein radiologisch oder sonologisch deutlich erkennbarer Ring (15) angeordnet ist.

## Claims

1. A device for introducing a two-component fibrin adhesive into a puncture channel into the vicinity of an arterial or venal puncture location, wherein this device comprises a sealing cannula (2) which is passed through axially from above to below by a working cannula which serves the intravascular introduction of an instrumentation into a vessel, wherein the working cannula (1) is surrounded by the sealing cannula (2) at a distance so that the fibrin adhesive is led from a connection piece (5) to at least one radially directed exit opening (6) in the sealing cannula between this and the working cannula, and wherein the sealing cannula (2) at the distal end is laterally formed on as one piece with a sleeve or wall thickening (4), **characterised in that** the sleeve (4) at the distal region comprises an outer thread (20) on which a medical coupling piece (3) with an inner thread is screwably held, said medical coupling piece comprising a distal introduction opening (30) and being rigidly connected in a flush manner to the working cannula which at the proximal end comprises inlet openings (11) which, by way of a relative movement of the medical coupling piece (3) to the sleeve (4) into a closure position, are movable into the sealing cannula (2).

2. A device according to claim 1, **characterised in that** the medical coupling piece (3) comprises a lateral control connection piece (31).

3. A device according to claim 2, **characterised in that** on the control connection piece (31) there is arranged a Luer-lock coupling (32).

4. A device according to claim 1, **characterised in that** on the medical coupling piece (3) there is attached a cover plate (39) in a distanced manner such that this plate covers the connection piece (5) when the inlet openings (11) are in the opening position, whilst it frees the connection piece (5) when the inlet openings (11) are in the closure position.

5. A device according to claim 1, **characterised in that** the medical coupling piece (3) comprises an inner thread (33) which meshes with an outer thread (20) on the sleeve (5) so that by rotating the medical coupling piece (3) to the sleeve (4) the inlet openings (11) are movable into the closure position within the sealing cannula (2) and into the opening position outside of the sealing cannula.

6. A device according to claim 1, **characterised in that** on the medical coupling piece there is formed an actuation lever (34).

7. A device according to claim 1, **characterised in that** at the distal end of the medical coupling piece (3) a lead-through seal (35) is held with a fixation (36).

8. A device according to claim 7,**characterised in that** the lead-through seal is simultaneously a haemostasis valve.

9. A device according to claim 1, **characterised in that** the distal end of the sealing cannula comprises two radially and inwardly directed annular sealing lips (12, 13) which are distanced from one another at least by the diameter of the inlet openings (11), and which sealingly bear on the working cannula (1) and which form an annular sealing chamber (14).

10. A device according to claim 1, **characterised in that** in the sealing chamber (14) there is arranged a radiologically or sonologically clearly recognisable ring (15).

## Revendications

1. Dispositif pour introduire de la colle à la fibrine à deux composants dans un canal de ponction à proximité d'un poste de ponction artériel ou veineux, auquel cas celui-ci comprend une canule d'obturation (2) qui est traversée axialement d'en haut vers le bas par une canule de travail (1) qui sert à l'introduction intravasculaire d'un instrument dans un vaisseau, auquel cas la canule de travail (1) est entouré de façon distancée par la canule d'obturation (2), de sorte que la colle à la fibrine est conduite à partir d'un tuyau de rallonge (5) au moins à un orifice de sortie (6), étant disposé radialement, dans la canule d'obturation entre celle-ci et la canule de travail, et auquel cas la canule d'obturation (2), à l'extrémité distale, est formée latéralement en tant qu'une seule pièce avec une manchette ou une compression de paroi (4), **caractérisé par le fait que** la manchette (4) présente sur la zone distale un filetage mâle (20) sur lequel une pièce d'accouplement médicale (3) avec filet femelle est maintenue par vis, laquelle présente un orifice d'entrée distal (30) et est solidement rattachée en alignement avec la canule de travail qui elle présente, à l'extrémité proximale, des orifices d'admission (11) qui sont susceptibles d'être déplacés à l'intérieur, par un mouvement relatif de la pièce d'accouplement médicale (3) vis-à-vis de la manchette (4), dans une position de verrouillage dans la canule d'obturation (2).

2. Dispositif selon la revendication 1, **caractérisé par le fait que** la pièce d'accouplement médicale (3) présente un raccord de contrôle (31) latéral.

3. Dispositif selon la revendication 2, **caractérisé par le fait qu'**un connecteur Luerlock (32) est placé sur le raccord de contrôle (31).

4. Dispositif selon la revendication 1, **caractérisé par le fait qu'**une plaque de recouvrement (39) est fixée à la pièce d'accouplement médicale (3) de sorte à être montée si en saillie qu'elle recouvre le raccord (5), si les orifices d'admission (11) se trouvent dans la position d'ouverture, tandis qu'elle libère le raccord (5), si les orifices d'admission (11) se trouvent dans la position fermée.

5. Dispositif selon la revendication 1, **caractérisé par le fait que** la pièce d'accouplement médicale (3) présente un filet femelle (38) qui se coince avec un filetage mâle (20) sur la manchette (5), de sorte que, par la torsion de la pièce d'accouplement médicale (3) par rapport à la manchette (4), les orifices d'admission (11) sont susceptibles d'être bougés dans la position fermée à l'intérieur de la canule d'obturation (2), ou respectivement dans la position d'ouverture à l'extérieur de la canule d'obturation.

6. Dispositif selon la revendication 1, **caractérisé par le fait qu'**un levier de manoeuvre (34) est reproduit sur la pièce d'accouplement médicale (3).

7. Dispositif selon la revendication 1, **caractérisé par le fait qu'**une garniture de traversée (35) est maintenue par une fixation (36) à l'extrémité distale de la pièce d'accouplement médicale (3).

8. Dispositif selon la revendication 7, **caractérisé par le fait que** la garniture de traversée (35) forme en même temps une valve hémostatique.

9. Dispositif selon la revendication 1, **caractérisé par le fait que** l'extrémité proximale de la canule d'obturation présente deux lèvres d'étanchéité circulaires (12, 13), étant distanciées l'une de l'autre au moins du diamètre des orifices d'admission (11) et étant dirigées radialement et vers l'intérieur et étant adjacentes de façon étanche à la canule de travail (1) et ces lèvres d'étanchéité forment une chambre d'étanchéité (14) circulaire.

10. Dispositif selon la revendication 1, **caractérisé par le fait qu'**un anneau clairement reconnaissable de façon radiologique ou sonologique (15) est placé de façon dans la chambre d'étanchéité.
